(19) **Europäisches Patentamt European Patent Office Office européen des brevets**

(11) **EP 3 040 092 B1**

(12) **FASCICULE DE BREVET EUROPEEN**

(45) Date de publication et mention de la délivrance du brevet:
**16.05.2018 Bulletin 2018/20**

(51) Int Cl.:
*F04B 43/04* (2006.01)  *F04B 43/12* (2006.01)
*F04B 53/10* (2006.01)  *A61F 13/00* (2006.01)
*A61M 1/00* (2006.01)  *F04B 43/073* (2006.01)

(21) Numéro de dépôt: **15202659.7**

(22) Date de dépôt: **24.12.2015**

(54) **POMPE SURFACIQUE ET PANSEMENT COMPORTANT UNE POMPE SURFACIQUE**

PUMPE UND WUNDBEHANDLUNGSVERBAND MIT EINER PUMPE

PUMP AND DRESSING HAVING A PUMP

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.12.2014 FR 1463432**

(43) Date de publication de la demande:
**06.07.2016 Bulletin 2016/27**

(73) Titulaire: **Commissariat à l'Énergie Atomique et aux Énergies Alternatives**
**75015 Paris (FR)**

(72) Inventeurs:
 • **FOUILLET, Yves**
  **38340 Voreppe (FR)**
 • **CONSTANTIN, Olivier**
  **38000 Grenoble (FR)**

(74) Mandataire: **Brevalex**
 **56, Boulevard de l'Embouchure**
 **B.P. 27519**
 **31075 Toulouse Cedex 2 (FR)**

(56) Documents cités:
 **WO-A2-02/082047  US-A1- 2008 275 409**
 **US-A1- 2014 134 002**

**Description**

**DOMAINE TECHNIQUE**

La présente invention concerne le domaine général de drainage microfluidique d'un liquide depuis une source surfacique et plus particulièrement, le drainage des exsudats sécrétés par une plaie. L'invention porte sur une pompe surfacique ainsi que sur un pansement pour des plaies exsudatives comportant une pompe surfacique.

**ETAT DE LA TECHNIQUE ANTERIEURE**

De manière générale, on utilise des canaux, des ventouses ou des matériaux poreux, reliés à des pompes pour aspirer un liquide sur une surface.

Plus particulièrement, pour drainer ou aspirer des liquides produits par des plaies, on utilise des systèmes de drainage à pression négative. En effet, les plaies aigües ou chroniques sécrètent des liquides appelés exsudats dont le contrôle est un problème important auquel sont confrontées les équipes soignantes. Ainsi, le traitement de la plaie consiste en partie à enlever les exsudats.

Le document WO 03/086232 décrit un exemple d'un système de traitement de plaies comprenant un pansement à pression négative. Le pansement comporte une couche en contact de la plaie, comprenant une zone centrale en communication avec une pompe à vide et une pluralité de canaux divergeant radialement de la zone centrale et comprenant des trous pour extraire l'exsudat de la plaie sous l'effet d'une pression négative.

Afin de créer une pression négative, le pansement doit nécessairement être étanche ce qui complique sa pose sur la plaie et demande une surveillance et un entretien continus. De plus, la pompe à vide est encombrante et bruyante.

En outre, la succion s'effectue de manière globale sur toute la surface du pansement. Ceci pose un problème de drainage au cas où certaines zones sont sèches et pas d'autres. En effet, l'effet de succion sera accentué sur la zone asséchée qui n'a pas besoin de drainage tout en empêchant le bon drainage des exsudats sécrétés par les autres zones.

Par ailleurs, l'exsudat est évacué vers une poche extérieure au pansement par une connectique fluidique qui augmente davantage l'encombrement du système. La connectique est bien entendu souillée par l'exsudat et peut éventuellement polluer la pompe.

L'objet de la présente invention est, par conséquent, de remédier aux inconvénients précités en proposant un système de pompage surfacique efficace avec une architecture de connexion simple et plus particulièrement, un pansement à pompage surfacique pour des plaies exsudatives, simple à poser, hygiénique et présentant un encombrement

Les documents WO02/082047A2, US2008/275409A1 divulguent des pompes micro-fluidiques à effet péristaltique.

**EXPOSÉ DE L'INVENTION**

L'invention a pour but de proposer une pompe surfacique compacte présentant une grande efficacité de drainage d'un liquide sur une surface quelle que soit l'hétérogénéité du taux d'humidité ou de l'étalement du liquide. Un autre but de l'invention est de présenter un pansement à pompage surfacique efficace et hygiénique pour des plaies exsudatives permettant une pose simplifiée, et présentant une architecture de connexion simple et peu encombrante.

L'invention a pour objet une pompe surfacique comportant un réseau de canaux microfluidiques et des moyens de pompage. Ledit réseau de canaux microfluidiques comprend un ensemble d'accès microfluidiques distribués dans un domaine prédéterminé adapté pour couvrir une source surfacique d'un fluide d'intérêt, et lesdits moyens de pompage comportent au moins un circuit microfluidique d'actionnement croisant ledit réseau de canaux microfluidiques sur une pluralité de zones de croisement à membrane déformable formant un ensemble de vannes adapté pour engendrer un pompage à effet péristaltique dudit fluide d'intérêt circulant dans ledit réseau de canaux microfluidiques depuis ledit ensemble d'accès microfluidiques vers au moins un puits connecté audit réseau de canaux microfluidiques.

Ainsi, la pompe surfacique est compacte, facile à mettre en oeuvre malgré que le nombre de vannes puisse être très élevé (par exemple de l'ordre de quelques centaines), et fonctionne de manière optimale pour pomper du liquide sur plusieurs voies pouvant couvrir une grande surface, et ceci quelle que soit l'hétérogénéité de l'étalement du liquide sur la surface.

Selon un mode de réalisation préféré de la présente invention, ledit réseau de canaux microfluidiques comporte un ensemble de canaux de drainage microfluidiques distincts muni dudit ensemble d'accès microfluidiques, chaque canal de drainage microfluidique étant associé à un groupe de vannes déterminé, et la pompe surfacique comporte un unique circuit microfluidique d'actionnement adapté pour être relié à un unique générateur de pression, ledit circuit d'actionnement étant constitué des éléments fluidiques résistifs et capacitifs définissant un retard de temps de réponse entre deux vannes successives appartenant à un groupe de vannes, ledit retard de temps de réponse étant configuré pour engendrer sous une action dudit générateur de pression, un actionnement séquentiel des vannes.

Ainsi, la pompe surfacique ne nécessite qu'une seule voie de connexion reliée à une seule source d'actionnement optimisant davantage sa compacité et sa simplicité.

Avantageusement, ledit ensemble de canaux de drainage microfluidiques comporte un ensemble de canaux microfluidiques principaux et une pluralité de canaux microfluidiques secondaires, chaque canal de drainage microfluidique comportant un canal microfluidique principal et un ensemble de canaux microfluidiques secondaires raccordant le canal principal aux accès microfluidiques associés.

Ceci permet d'homogénéiser et d'augmenter la densité des points d'accès pour un drainage optimal.

Selon un premier mode de réalisation préféré de la présente invention, ledit circuit microfluidique d'actionnement est formé d'un canal microfluidique d'actionnement en forme de serpentin, dit serpentin d'actionnement, présentant une longueur caractéristique $L$ entre deux vannes successives appartenant à un même groupe de vannes, une largeur $w$,

une hauteur $h$, et un paramètre de déformation $A$, vérifiant la double inégalité suivante : $1 \times 10^{-3} \leq A\frac{wL^2}{h^3} \leq 10$

et de préférence $0{,}01 \leq A\frac{wL^2}{h^3} \leq 1$.

Ceci permet de créer de manière simple un gradient de pression transitoire entre toutes les deux vannes successives d'un même groupe de vannes engendrant de manière efficace un effet de pompage péristaltique sur chaque canal de drainage microfluidique et quelle que soit la longueur du canal.

Selon un deuxième mode de réalisation préféré de la présente invention, ledit circuit microfluidique d'actionnement comporte des canaux d'actionnement en forme de peigne définissant des éléments capacitifs, raccordés à un canal de raccordement définissant un élément résistif, les vannes étant localisées sur lesdits canaux d'actionnement.

Avantageusement, des extrémités avals desdits canaux microfluidiques principaux sont connectées à un même puits, les autres extrémités étant fermées.

Selon une variante, l'ensemble de canaux microfluidiques principaux est constitué des premier et second sous-ensembles distincts de canaux microfluidiques principaux, le premier sous-ensemble de canaux microfluidiques principaux étant connecté à un premier puits et le second sous-ensemble de canaux microfluidiques principaux étant connecté à un second puits.

Avantageusement, ledit générateur de pression est configuré pour générer des créneaux de pression pour actionner de manière cyclique les vannes appartenant à chaque groupe de vannes.

Selon un troisième mode de réalisation préféré de la présente invention, ledit réseau de canaux microfluidiques comporte un canal microfluidique principal en forme de spirale raccordé via des canaux microfluidiques secondaires audit ensemble d'accès microfluidiques, et la pompe surfacique comporte au moins trois circuits microfluidiques d'actionnement distincts adaptés pour être relié à au moins trois générateurs de pression distincts, chaque circuit d'actionnement étant constitué d'un canal d'actionnement en forme de spirale, lesdits au moins trois canaux d'actionnement coopérant avec chaque canal microfluidique secondaire pour former au moins trois vannes configurées pour être actionnées de manière séquentielle par lesdits au moins trois générateurs de pression.

Avantageusement, la pompe surfacique comporte un premier substrat, une membrane déformable, et un second substrat, ledit au moins un circuit d'actionnement étant délimité par ladite membrane déformable et ledit premier substrat, ledit réseau de canaux microfluidiques étant délimité par ladite membrane déformable et ledit second substrat, ledit réseau de canaux microfluidiques et ledit au moins un circuit d'actionnement étant alors séparés par ladite membrane déformable aux niveaux des zones de croisement formant ainsi une vanne à chaque croisement.

Avantageusement, la pompe surfacique comporte un nombre déterminé de vannes compris entre environ 10 et 1000.

L'invention porte également sur un système de pompage comportant une pompe surfacique selon l'une quelconque des caractéristiques précédentes, comportant en outre au moins un générateur de pression relié audit au moins un circuit d'actionnement de ladite pompe surfacique.

L'invention vise aussi un pansement comportant une pompe surfacique selon l'une quelconque des caractéristiques précédentes, comportant au moins un puits connecté à ladite pompe surfacique, ledit au moins un puits étant destiné à collecter le fluide d'intérêt pompé par la pompe surfacique.

L'invention porte également sur un pansement pour des plaies exsudatives comportant un ensemble de canaux de drainage microfluidiques munis d'une pluralité d'accès microfluidiques définissant un domaine prédéterminé adapté pour couvrir une plaie sécrétant de l'exsudat, un circuit microfluidique d'actionnement adapté pour être relié à un unique générateur de pression, ledit circuit microfluidique d'actionnement croisant lesdits canaux de drainage microfluidiques sur une pluralité de zones de croisement à membrane déformable formant un ensemble de vannes adapté pour engendrer sous un actionnement dudit générateur de pression, un pompage à effet péristaltique de l'exsudat circulant dans lesdits canaux de drainage microfluidiques depuis ledit ensemble d'accès microfluidiques vers au moins un puits connecté audit ensemble de canaux de drainage microfluidiques.

Ainsi, contrairement à la technique de pression négative, le drainage est intégré au pansement de manière localisée et non globale. De plus, il n'est pas nécessaire de rendre le pansement étanche, ce qui simplifie sa pose, sa surveillance et son entretien. En outre, le pansement possède un encombrement réduit avec une seule voie de connexion reliée à un seul générateur de pression externe pour pomper l'exsudat sécrété sur toute la surface de la plaie. Par ailleurs, la connectique au pansement n'est pas en contact avec les exsudats et ainsi le générateur de pression externe n'est pas souillée par les exsudats et peut être réutilisé. Un autre avantage est le fait que le générateur de pression utilisé par le pansement est non encombrant, portable et présente une technologie très simple à mettre en oeuvre.

Selon un premier mode de réalisation préféré du pansement, le circuit microfluidique d'actionnement est formé d'un canal microfluidique d'actionnement en forme de serpentin.

Selon un deuxième mode de réalisation préféré du pansement, ledit circuit microfluidique d'actionnement comporte des canaux d'actionnement en forme de peigne définissant des éléments capacitifs, raccordés à au moins un canal de raccordement définissant un élément résistif, les vannes étant localisées sur lesdits canaux d'actionnement.

Avantageusement, ledit ensemble de canaux de drainage microfluidiques comporte un ensemble de canaux microfluidiques principaux et une pluralité de canaux microfluidiques secondaires, chaque canal de drainage microfluidique comportant un canal microfluidique principal et un ensemble de canaux microfluidiques secondaires raccordant le canal principal aux accès microfluidiques associés.

Avantageusement, le pansement comporte des premier et deuxième puits pour collecter les exsudats sécrétés par la plaie et ledit ensemble de canaux microfluidiques principaux est constitué des premier et second sous-ensembles distincts de canaux microfluidiques principaux, le premier sous-ensemble de canaux microfluidiques principaux étant connecté au premier puits et le second sous-ensemble de canaux microfluidiques principaux étant connecté au second puits.

Ainsi, les puits sont intégrés au pansement facilitant encore davantage la pose et supprimant les connexions à une poubelle externe.

Avantageusement, l'ensemble de canaux de drainage microfluidiques et le canal fluidique d'actionnement sont réalisés en matière biocompatible.

Ainsi, ces canaux peuvent être déposés directement sur la plaie.

Avantageusement, le pansement comporte une couche support accueillant l'ensemble de canaux de drainage microfluidiques et le canal fluidique d'actionnement, ladite couche support étant formée d'un matériau poreux biocompatible destiné à être en contact avec la plaie.

Avantageusement, le pansement comporte une couche externe autocollante.

Ceci permet de protéger la plaie et de faciliter la pose du pansement sur la plaie.

D'autres avantages et caractéristiques de l'invention apparaîtront dans la description détaillée non limitative ci-dessous.

## BRÈVE DESCRIPTION DES DESSINS

On décrira à présent, à titre d'exemples non limitatifs, des modes de réalisation de l'invention, en se référant aux dessins annexés, dans lesquels :

La Fig. 1 illustre de manière très schématique une pompe surfacique, selon l'invention ;
La Fig. 2 illustre de manière schématique une pompe surfacique, selon un mode de réalisation préféré de l'invention ;
La Fig. 3 illustre de manière schématique une pompe surfacique, selon un premier mode de réalisation préféré de l'invention ;
La Fig. 4 est un exemple expérimental d'un système de pompage illustrant l'action d'un unique circuit microfluidique d'actionnement sur un canal microfluidique, selon le mode de réalisation de la Fig. 3 ;
La Fig. 5 illustre de manière schématique une pompe surfacique, selon un deuxième mode de réalisation préféré de l'invention ;
La Fig. 6 illustre de manière schématique une pompe surfacique, selon un troisième mode de réalisation préféré de l'invention ;
Les Figs. 7A-7F illustrent de manière schématique, un procédé de fabrication d'une pompe surfacique, selon l'invention ;
La Fig. 8 illustre de manière très schématique un système de pompage selon un mode de réalisation de l'invention ;
La Fig. 9 illustre de manière schématique un pansement comportant une pompe surfacique, selon un mode de réalisation préféré de l'invention ; et
Les Figs. 10A et 10B illustrent de manière schématique un pansement selon des premier et deuxième modes de réalisation préférés de l'invention.

## EXPOSÉ DÉTAILLÉ D'UN MODE DE RÉALISATION PRÉFÉRÉ

Le concept à la base de l'invention consiste à réaliser un système de drainage surfacique à partir d'un réseau de micropompes péristaltiques et de l'appliquer à un pansement pour des plaies exsudatives.

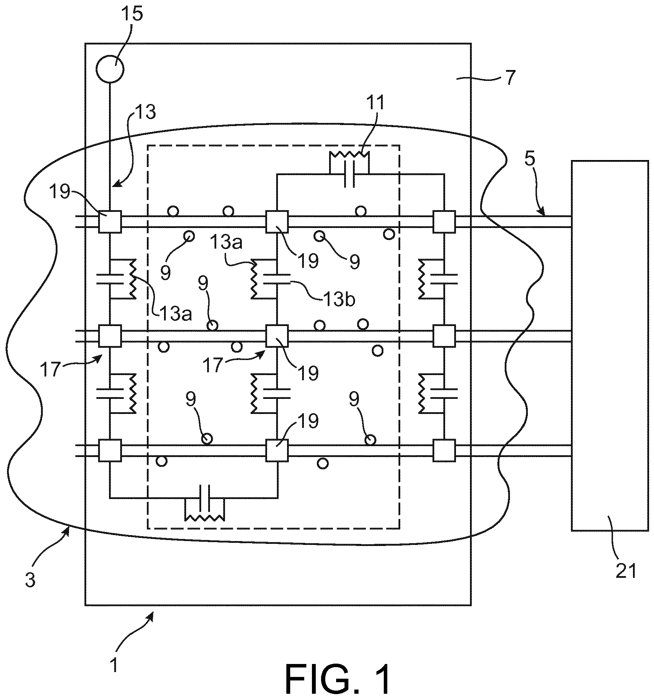

La Fig. 1 illustre de manière très schématique une pompe surfacique, selon l'invention.

La pompe surfacique 1 comporte un réseau 3 de canaux microfluidiques 5 transportant un liquide d'intérêt à drainer, et des moyens de pompage 7 pour actionner le pompage dans ces canaux microfluidiques.

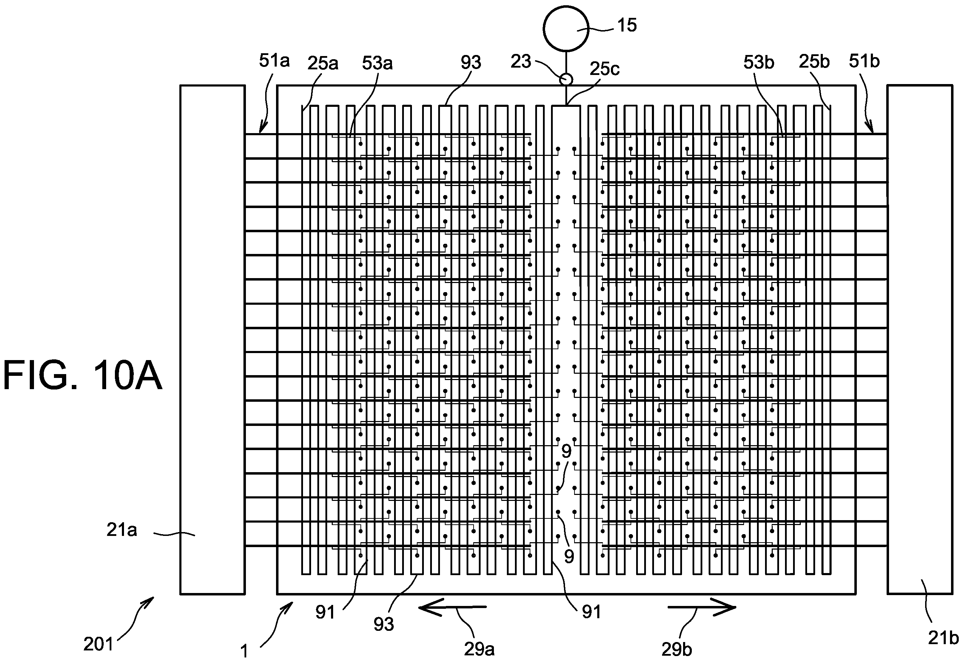

Le réseau 3 de canaux microfluidiques 5 comprend un ensemble d'accès 9 microfluidiques disposés dans un domaine 11 prédéterminé adapté pour couvrir une source surfacique du fluide d'intérêt. Les accès 9 microfluidiques peuvent être distribués de manière homogène et planaire dans le domaine 11 prédéterminé. La frontière du domaine 11 prédéterminé peut être de forme géométrique quelconque (par exemple, rectangulaire, circulaire, ovale) qui s'adapte à la forme de la source surfacique du fluide. A titre d'exemple, les accès 9 microfluidiques sont disposés de manière matricielle (voir par exemple Fig. 10A).

Les moyens de pompage 7 comportent au moins un circuit microfluidique d'actionnement 13 représenté très schématiquement sur la Fig. 1 par un modèle rhéologique. Plus particulièrement, les éléments fluidiques d'actionnement constituant chaque circuit microfluidique d'actionnement sont modélisés par des éléments fluidiques résistifs 13a et capacitifs 13b définis par des grandeurs fluidiques de résistance et de capacité.

Chaque circuit d'actionnement 13 est adapté pour être relié à un générateur de pression 15 et contient un fluide de viscosité déterminé, sélectionné parmi un ensemble de fluides comportant par exemple, de l'eau, de l'air, de l'huile, ou autre types de fluides selon l'application.

Les éléments fluidiques d'actionnement sont géométriquement et matériellement configurés pour permettre à chaque circuit d'actionnement 13 de croiser le réseau 3 de canaux microfluidiques 5 sur une pluralité de noeuds ou zones de croisement 17 à membrane déformable formant un réseau ou ensemble de vannes 19 réparties au sein du réseau 3 de canaux microfluidiques. Chaque vanne 19 est formée par le croisement (i.e. la superposition) d'un élément fluidique d'actionnement avec un canal microfluidique 5 correspondant. Ainsi, une vanne 19 appartenant à un canal microfluidique 5 est adaptée pour comprimer et fermer ce canal, partiellement ou en totalité sous la mise en pression de l'élément fluidique d'actionnement coopérant avec le canal microfluidique.

L'ensemble de vannes 19 est adapté pour engendrer sous l'actionnement contrôlé du ou des générateur(s) de pression 15 un pompage du fluide d'intérêt circulant dans le réseau 3 de canaux microfluidiques. En effet, la membrane au niveau de chaque vanne 19 applique une action mécanique au fluide dans le canal microfluidique correspondant, provoquant le déplacement du fluide dans ce canal. L'actionnement successif des vannes 19 appartenant à un même canal microfluidique 5 génère un effet de micropompage appelé péristaltique. Autrement dit, le groupe de vannes 19 appartenant à chaque canal microfluidique 5 forme un ensemble de micropompes péristaltiques coordonnées entre elles pour créer tout au long du canal un micropompage péristaltique du fluide qui s'introduit dans le canal microfluidique via les différents accès 9 microfluidiques. Ainsi, le réseau 3 de canaux microfluidiques 5 forme un réseau de micropompes.

Le document FR2974598 décrit un exemple d'une micropompe péristaltique en silicium comportant trois membranes déformables actionnées par trois éléments piézoélectriques distincts. D'autres exemples sont décrits dans la publication « Monolithic microfabricated Valves and Pumps by Multilayer Soft Lithography », Marc Unger et al. Science 288, 113 (2000). Ces exemples concernent en général des micropompes linéiques comportant un seul canal microfluidique transportant le liquide et trois canaux pneumatiques pilotés de manière séquentielle par trois actionneurs.

Toutefois, selon un aspect de l'invention, la pompe surfacique 1 est constituée d'un réseau de micropompes péristaltiques drainant un fluide d'intérêt sur plusieurs voies affluant depuis un ensemble ou une matrice d'accès 9 microfluidiques pouvant couvrir une grande surface.

Ainsi, les vannes 19 contrôlent la circulation du fluide d'intérêt dans le réseau 3 de canaux microfluidiques 5 pour engendrer un pompage à effet péristaltique du fluide depuis l'ensemble d'accès 9 microfluidiques vers au moins un puits 21 connecté au réseau 3 de canaux microfluidiques 5.

La pompe surfacique selon l'invention peut être utilisée dans de nombreux systèmes fluidiques tels que, à titre d'exemples, des laboratoires sur puce, ou encore des circuits hydrauliques de refroidissement de puces électroniques.

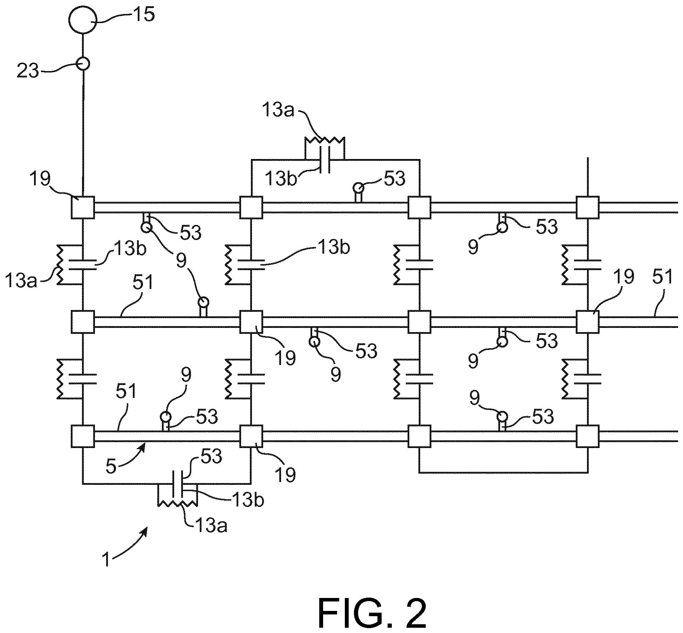

La Fig. 2 illustre de manière schématique une pompe surfacique, selon un mode de réalisation préféré de l'invention.

Selon ce mode de réalisation, le réseau de canaux microfluidiques comporte un ensemble de canaux de drainage microfluidiques 5 distincts muni d'un ensemble d'accès 9 microfluidiques.

L'ensemble de canaux de drainage microfluidiques 5 peut par exemple comporter un ensemble de canaux microfluidiques principaux 51 et une pluralité de canaux microfluidiques secondaires 53. Dans ce cas, chaque canal de drainage microfluidique 5 comporte un canal microfluidique principal 51 et un ensemble de canaux microfluidiques secondaires 53 raccordant le canal principal 51 aux accès 9 microfluidiques associés.

En outre, chaque canal de drainage microfluidique 5 est associé à un groupe de vannes déterminé avec au moins une vanne 19 (et de préférence plusieurs vannes) par canal secondaire 53. Le dessin montre trois canaux de drainage microfluidiques 5 avec un groupe de quatre vannes 19 par canal principal 51 disposées de sorte qu'il y ait une vanne par canal secondaire 53, mais bien entendu, l'invention peut s'appliquer à une pompe surfacique comportant un nombre quelconque de canaux de drainage microfluidiques 5 ainsi qu'un nombre quelconque de vannes 19 par groupe et de plus, différents groupes peuvent comporter des nombres différents de vannes.

Une particularité avantageuse de ce mode de réalisation est que la pompe surfacique 1 comporte un unique circuit microfluidique d'actionnement 13 adapté pour être relié à un unique générateur de pression 15 via un unique port de connexion (ou entrée) 23. Ce circuit d'actionnement 13 est constitué des éléments fluidiques résistifs 13a et capacitifs 13b d'actionnement définissant un retard de temps de réponse entre deux vannes 19 successives appartenant à un même groupe de vannes (i.e. sur un même canal de drainage microfluidique 51). Le retard de temps de réponse est créé par le fait que les éléments fluidiques résistifs et capacitifs d'actionnement génèrent un gradient de pression transitoire entre chaque deux vannes 19 successives d'un canal de drainage microfluidique 51.

Le retard de temps de réponse est configuré pour engendrer sous une action du générateur de pression 15, un actionnement séquentiel des vannes 19 appartenant à chaque groupe de vannes créant alors un pompage péristaltique dans le canal de drainage microfluidique 51.

En outre, le générateur de pression 15 est configuré pour générer des créneaux de pression permettant d'actionner les vannes 19 appartenant à chaque groupe de vannes 19 de manière cyclique. On notera qu'un cycle correspond au front de propagation de l'ouverture puis de la fermeture des vannes 19. Ainsi, quand une pression haute est appliquée au circuit microfluidique d'actionnement 13, les vannes 19 de chaque groupe de vannes passent d'abord d'un état ouvert à un état fermé les unes après les autres grâce au retard de temps de réponse et ensuite, quand une pression basse est appliquée au circuit fluidique, les vannes 19 de chaque groupe de vannes passent de l'état fermé à un état ouvert les unes après les autres.

Le retard de temps de réponse d'actionnement entre deux vannes 19 successives $V_{n-1}$, $V_n$ d'un canal de drainage microfluidique 51 est déterminé par les valeurs de résistance et de capacité des différents éléments fluidiques 13a, 13b entre ces deux vannes. On notera que pour des écoulements monophasiques, l'équivalence électrique d'un circuit microfluidique est possible grâce à une linéarisation de l'équation de Stokes.

Ainsi, le retard de temps de réponse peut s'exprimer par une constante de temps $\tau$ définie par le produit $\tau = R \times C$ entre la résistance fluidique $R$ et la capacité fluidique $C$ relatives aux deux vannes 19 successives.

La notion de résistance fluidique $R$ est liée à la dissipation d'énergie et peut s'exprimer comme le rapport entre la perte de charge $\Delta P$ et le débit $Q$ à travers un canal microfluidique.

La capacité fluidique $C$ peut se définir par la dilatation volumique $\delta V$ d'un canal microfluidique à la suite d'une augmentation de pression $\delta P$.

Avantageusement, les valeurs des résistances et capacités fluidiques sont choisies pour que le retard de temps de réponse $\tau$ soit compris entre une milliseconde et une minute et de préférence, entre environ 0,01 seconde et quelques secondes.

Les valeurs des résistances et capacités fluidiques sont déterminées par le dimensionnement et la composition matérielle du circuit d'actionnement 13.

En effet, la résistance fluidique $R$ d'un canal microfluidique est fonction de sa longueur caractéristique $L$, sa largeur $w$, sa hauteur $h$, un paramètre $K$ dépendant de la forme de sa section, ainsi que de la viscosité $\mu$ du fluide contenu dans le canal. Plus particulièrement, la résistance fluidique $R$ est définie par l'expression suivante :

$$R = \frac{K\mu L}{wh^3} \quad (1)$$

le paramètre $K$ est en général compris entre 12 et 32 et en particulier, $K = 32$ pour un canal de section carré et $K = 12$ pour un canal de section rectangulaire avec $h < w$.

La capacité fluidique $C$ peut s'exprimer en fonction des dimensions du canal, de son module de Young $E$ et d'un paramètre $\alpha$ qui dépend du matériau constituant le canal, selon l'expression suivante :

$$C = \alpha \frac{Lw^2}{E} \quad (2)$$

En particulier, les équations (1) et (2) indiquent qu'en augmentant la largeur $w$ d'un canal, on diminue sa résistance fluidique $R$ tout en augmentant sa capacité $C$ et inversement. Ainsi, on peut par exemple augmenter la largeur $w$ des canaux aux niveaux des croisements pour augmenter la dilatation et l'efficacité des vannes.

Ainsi, chaque tronçon de canal d'actionnement entre deux croisements peut être assimilé à un circuit élémentaire $R_iC_i$ en parallèle. Cela explique qu'en appliquant une variation de pression à l'entrée 23 du canal d'actionnement 13, cette variation de pression se propage entre deux vannes successives du circuit d'actionnement 13 selon un temps de réponse $\tau_i = R_i \times C_i$. D'une façon générale, le circuit d'actionnement comporte une série de vannes $V_1,...,V_n,...V_N$. Chaque vanne $V_n$ est caractérisée par un temps de réponse $\tau_n = R_n \times C_n$, $R_n$ et $C_n$ caractérisant le circuit d'actionnement entre la vanne $V_n$ et l'entrée 23 du circuit d'actionnement 13. Plus la distance entre une vanne $V_n$ et l'entrée 23 du circuit 13 est élevée, plus le temps de réponse $\tau_n$ augmente.

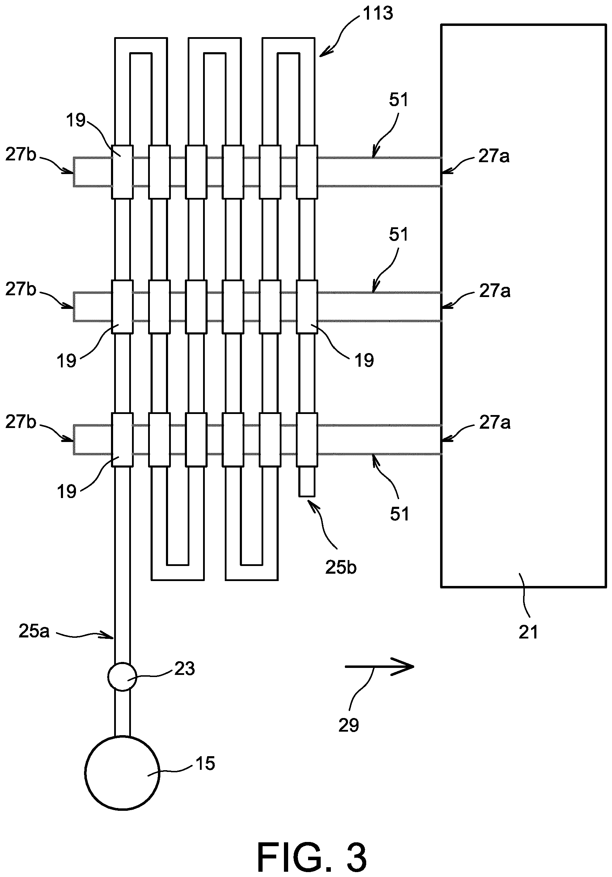

La Fig. 3 illustre de manière schématique une pompe surfacique, selon un premier mode de réalisation préféré de l'invention. Par souci de simplification, les accès microfluidiques ne sont pas illustrés sur le dessin de la Fig. 3.

Selon ce premier mode de réalisation, le circuit microfluidique d'actionnement est formé d'un canal microfluidique d'actionnement 113 en forme de serpentin, dit serpentin d'actionnement 113.

L'exemple illustré sur la Fig. 3 montre qu'une extrémité 25a du serpentin d'actionnement 113 est connectée via l'unique port de connexion 23 au générateur de pression 15, tandis que l'autre extrémité 25b est fermée. Par ailleurs, les extrémités avals 27a par rapport au sens de pompage (indiqué par la flèche 29) des canaux microfluidiques principaux 51 sont connectées à un même puits 21, les autres extrémités 27b étant fermées.

Selon une variante (voir l'exemple de la Fig. 10A), l'ensemble de canaux microfluidiques principaux 51 est constitué des premier et second sous-ensembles distincts de canaux microfluidiques principaux 51a, 51b. Le premier sous-ensemble de canaux microfluidiques principaux 51a est connecté à un premier puits 21a et le second sous-ensemble de canaux microfluidiques principaux 51b est connecté à un second puits 21b. Dans ce cas, les extrémités du serpentin d'actionnement 25a, 25b sont fermées et un point intermédiaire 25c entre ces deux extrémités est connecté au générateur de pression 15 (Fig. 10A).

Selon l'une ou l'autre variante, le serpentin d'actionnement 113 présente une longueur caractéristique $L$ entre par exemple deux vannes 19 successives appartenant à un même groupe de vannes, une largeur $w$, et une hauteur $h$.

En utilisant, les équations (1) et (2) ci-dessus, le retard de temps de réponse $\tau$ est donné par l'expression suivante :

$$\tau = RC = \frac{K\mu L}{wh^3} \times \frac{\alpha L w^2}{E} = A\frac{wL^2}{h^3} \quad (3)$$

où $A = \frac{K\alpha\mu}{E}$ est un coefficient ou paramètre de déformation qui dépend du module de Young $E$ du matériau du serpentin d'actionnement 113 et du coefficient de viscosité $\mu$ du fluide contenu dans le serpentin d'actionnement 113.

Avantageusement, le matériau et dimensions du serpentin d'actionnement 113 sont sélectionnés de manière à vérifier la double inégalité suivante : $1 \times 10^{-3} \le A\frac{wL^2}{h^3} \le 10,$ et de préférence $0,01 \le A\frac{wL^2}{h^3} \le 1.$

À titre d'exemple, pour une pompe surfacique réalisée en PDMS composé d'un mélange de dix volumes de polymère pour un volume de réticulant, le module de Young est $E = 2,5 \times 10^6$ $Pa$, le paramètre $\alpha$ est pratiquement égal à 0,5 et pour un serpentin d'actionnement 113 de section rectangulaire rempli d'eau, la viscosité est $\mu = 0,001$ $Pa.s$ et $K$ est un peu plus grand que 12. Dans ce cas, le paramètre de déformation $A$ est de l'ordre de $3 \times 10^{-9}$. Par exemple avec un serpentin d'actionnement 113 de largeur $w = 65$ $\mu m$ et une hauteur $h = 10$ $\mu m$, il suffit d'avoir une longueur caractéristique $L$ entre deux vannes 19 successives de l'ordre de 3 cm pour viser un temps de réponse $\tau$ de l'ordre de 0,1 $s$.

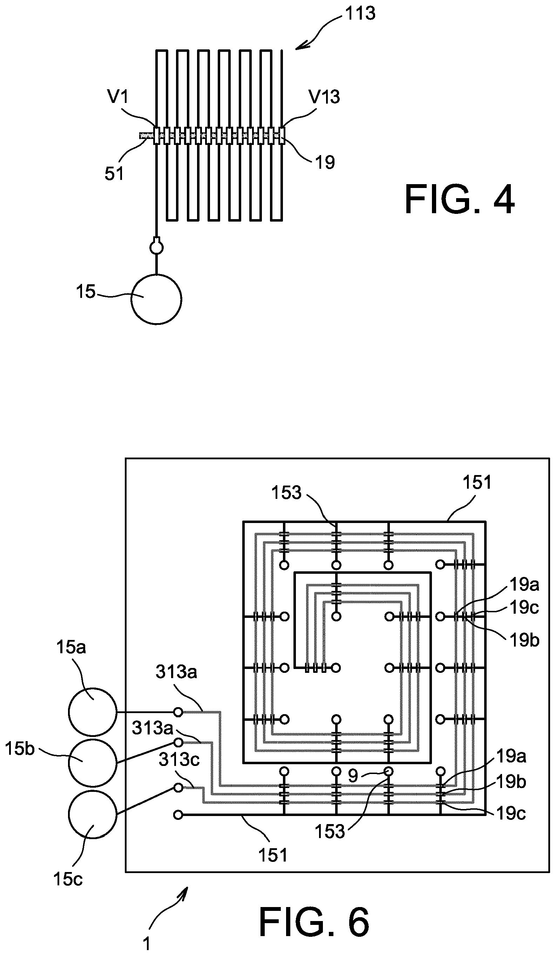

La Fig. 4 est un exemple expérimental d'un système de pompage illustrant l'action d'un unique circuit microfluidique d'actionnement sur un canal microfluidique, selon le mode de réalisation de la Fig. 3.

Selon cet exemple illustratif, le serpentin d'actionnement 113 croise le canal de drainage microfluidique pour former une suite de 13 vannes $V_1,...,V_{13}$ à un pas de 400 $\mu m$. Le serpentin d'actionnement 113 présente une longueur totale, entre les vannes $V_1$ et $V_{13}$ de 90 $mm$, une largeur $w$ de 65 $\mu m$, et une hauteur $h = 10$ $\mu m$ (selon une première version du système de pompage) et une hauteur $h = 50$ $\mu m$ (selon une deuxième version du système de pompage). La largeur du serpentin au niveau des vannes est de 200 $\mu m$. Le canal de drainage microfluidique présente une largeur de 200 $\mu m$ et la surface totale du système de pompage est d'environ 6 $mm \times 6$ $mm$.

Un générateur de pression 15 générant des créneaux de pression d'amplitude $P_- = -100$ $mbar$ à $P_+ = 600$ $mbar$ à une fréquence déterminée est connecté au serpentin d'actionnement 113 du système de pompage. On notera qu'un cycle correspond au front de propagation de l'ouverture puis de la fermeture des vannes.

Selon la première version ($h = 10$ $\mu m$), la fréquence du générateur de pression est choisie égale à 0,1 $Hz$. Le débit mesuré est de l'ordre de 0,8 $nl$ par cycle soit environ 4 $nl/mn$. Le retard de temps de réponse total observé entre la première vanne $V_1$ et la dernière vanne $V_{13}$ est entre 1,2 s et 4,4 s. La valeur de 1,2s est observée lorsqu'on applique une surpression, ce qui entraîne la fermeture des vannes successives, tandis que la valeur de 4,4s est mesurée lorsqu'on applique une dépression, ce qui entraîne l'ouverture des vannes successives. Cette différence résulte d'effets non linéaires non pris en compte dans le modèle de la présente demande. On notera que le modèle théorique pour la première version ($h$ = 10 $\mu m$) prédit un retard de temps de réponse total entre la première vanne V$_1$ et la dernière vanne V$_{13}$ de 1,3 s.

Selon la deuxième version ($h$ = 50 $\mu m$), la fréquence du générateur de pression est comprise entre 0,5 $Hz$ et 4 $Hz$. Le débit mesuré à une fréquence de 1 $Hz$ est de l'ordre de 0,6 $\mu l/mn$ soit environ 10 $nl$ par cycle. Le débit mesuré est de l'ordre de 1 $\mu l/mn$ à une fréquence de 2 $Hz$. Le retard de temps de réponse total observé entre la première vanne V$_1$ et la dernière vanne V$_{13}$ est de l'ordre de 0,13 s. On notera que le modèle théorique pour la deuxième version du système de pompage prédit un retard de temps de réponse total entre la première vanne V$_1$ et la dernière vanne V$_{13}$ de 0,03 s.

Les valeurs expérimentales des première et deuxième versions montrent une bonne corrélation (moins d'une décade) entre le modèle théorique et l'expérience, compte tenu des hypothèses assez simples de modélisation.

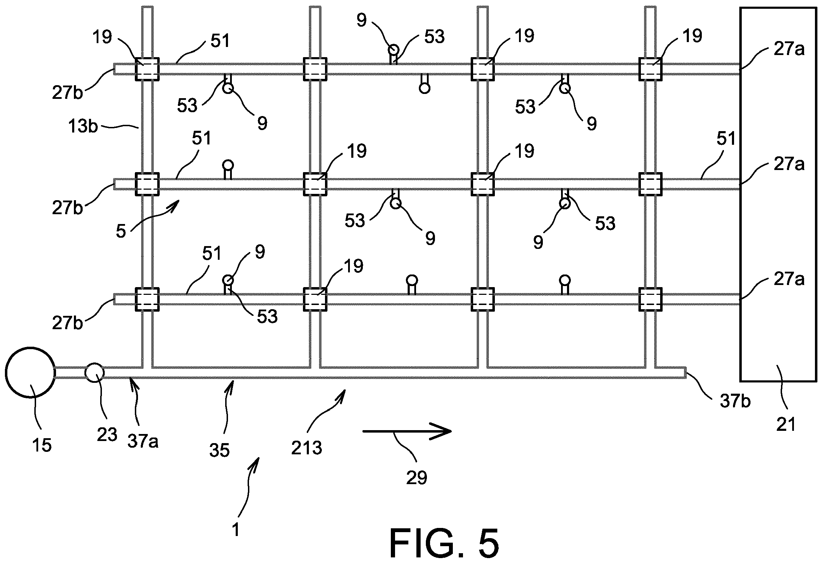

La Fig. 5 illustre de manière schématique une pompe surfacique, selon un deuxième mode de réalisation préféré de l'invention.

De manière similaire au mode de réalisation précédent, le réseau de canaux microfluidiques comporte un ensemble de canaux de drainage microfluidiques 5 distincts muni d'un ensemble d'accès 9 microfluidiques. L'ensemble de canaux de drainage microfluidiques 5 comporte un ensemble de canaux microfluidiques principaux 51 et une pluralité de canaux microfluidiques secondaires 53. Chaque canal de drainage microfluidique 5 comporte un canal microfluidique principal 51 et un ensemble de canaux microfluidiques secondaires 53 raccordant le canal principal aux accès 9 microfluidiques associés.

En outre, chaque canal de drainage microfluidique 5 est associé à un groupe de vannes déterminé. Le dessin montre trois canaux de drainage microfluidiques 5 avec un groupe de quatre vannes 19 par canal.

Par ailleurs, la pompe surfacique 1 comporte un unique circuit microfluidique d'actionnement 213. Toutefois, selon ce deuxième mode de réalisation, l'unique circuit microfluidique d'actionnement 213 comporte des canaux d'actionnement 33 en forme de peigne formant principalement des éléments capacitifs. Les vannes 19 sont localisées sur les canaux d'actionnement 33 et ses derniers sont raccordés à un canal de raccordement 35 qui forme principalement un élément résistif. Les canaux d'actionnement 33 sont par exemple sensiblement perpendiculaires au canal de raccordement 35. Le fait que les canaux d'actionnement 33 présentent des résistances fluidiques très faibles devant la résistance fluidique du canal de raccordement 35 permet de créer un retard de temps de réponse d'actionnement entre deux vannes 19 successives appartenant à un même groupe de vannes, c'est-à-dire disposées le long d'un même canal microfluidique principal. Plus précisément, le retard de temps de réponse dépend comme indiqué précédemment, de la capacité de dilatation de chaque canal d'actionnement 33 et de la résistance fluidique à l'établissement de l'écoulement pour une pression donnée dans le canal d'actionnement.

Une extrémité 37a du canal de raccordement 35 peut être connectée via un unique port de connexion 23 à un unique générateur de pression 15. L'autre extrémité 37b du canal de raccordement 35 ainsi que les extrémités libres 37c des canaux d'actionnement 33 sont fermées.

Le retard de temps de réponse est configuré pour engendrer sous une action du générateur de pression 15, un actionnement séquentiel des vannes 19 appartenant à chaque groupe de vannes créant alors un pompage péristaltique. En outre, le générateur de pression 15 est configuré pour générer des créneaux de pression permettant d'actionner les vannes appartenant à chaque groupe de vannes de manière cyclique.

Avantageusement, les extrémités 27a avals (par rapport au sens de pompage 29) des canaux microfluidiques principaux 51 sont connectées à un même puits 21 tandis que les autres extrémités 27b sont fermées. Selon cette variante, les vannes de chaque canal microfluidique principal sont mieux synchronisées. On estime que deux vannes adjacentes, situées sur deux canaux voisins, seront simultanément activées.

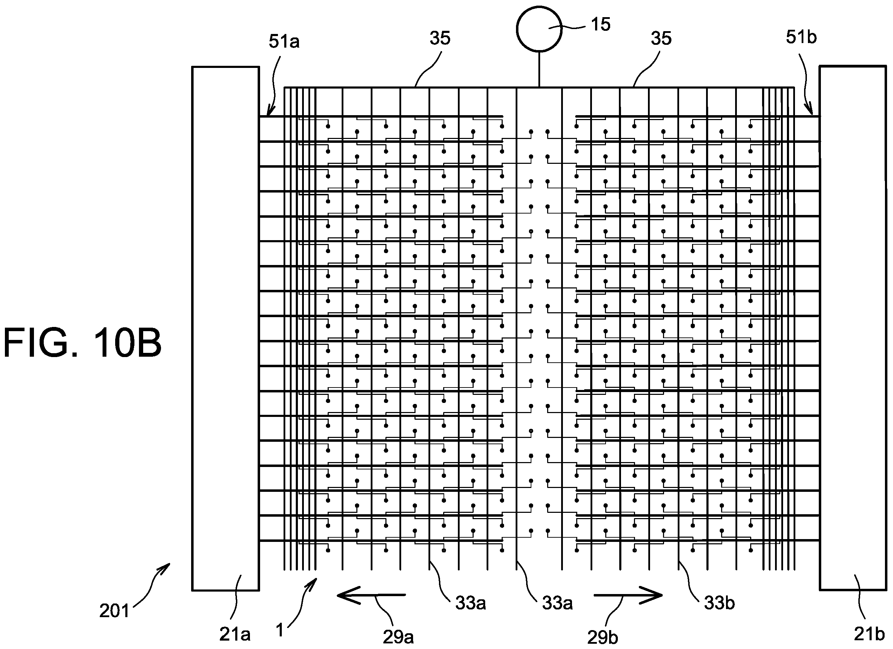

Selon une variante (voir l'exemple de la Fig. 10B), l'ensemble de canaux microfluidiques principaux 51 est constitué des premier et second sous-ensembles distincts de canaux microfluidiques principaux 51a, 51b. Le premier sous-ensemble de canaux microfluidiques principaux 51a est connecté à un premier puits 21a et le second sous-ensemble de canaux microfluidiques principaux 51b est connecté à un second puits 21b. Dans ce cas, deux canaux d'actionnement 33a, 33b situés environ au milieu du circuit d'actionnement 213 sont connectés à un même générateur de pression 15.

La Fig. 6 illustre de manière schématique une pompe surfacique, selon un troisième mode de réalisation préféré de l'invention.

Selon ce mode de réalisation, le réseau de canaux microfluidiques comporte un unique canal microfluidique principal 151 en forme de spirale, raccordé via des canaux microfluidiques secondaires 153 à un ensemble d'accès 9 microfluidiques.

En outre, la pompe surfacique 1 comporte au moins trois circuits microfluidiques d'actionnement 313a, 313b, 313c distincts adaptés pour être reliés à au moins trois générateurs de pression 15a, 15b, 15c distincts. Chaque circuit d'actionnement 313a, 313b, 313c est constitué d'un canal d'actionnement en forme de spirale semblable à celle du canal microfluidique principal 151. Ainsi, les circuits d'actionnement 313a, 313b, 313c jouxtent le canal microfluidique principal 151 et coopèrent avec chaque canal secondaire 153 de drainage pour former un groupement d'au moins trois vannes 19a, 19b, 19c. Les vannes appartenant à chaque groupement de vannes 19a, 19b, 19c sont configurées pour être actionnées de manière séquentielle par les générateurs de pression 15a, 15b, 15c.

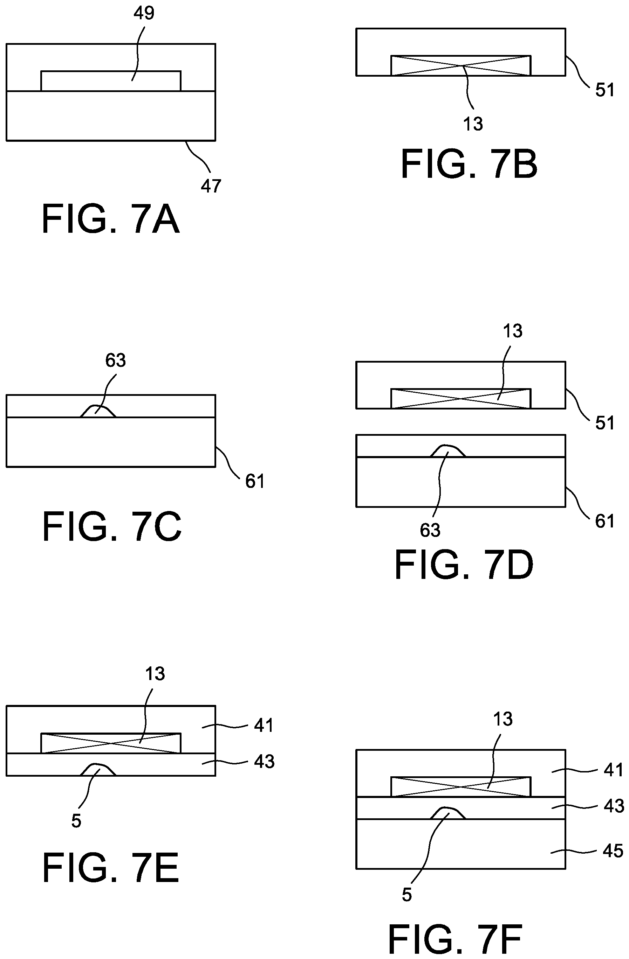

Les Figs. 7A-7F illustrent de manière schématique, un procédé de fabrication d'une pompe surfacique, selon l'invention.

La pompe surfacique selon les différents modes de réalisation de l'invention comporte un premier substrat 41, une membrane déformable 43, et un second substrat 45 (voir Fig. 7F). Le (ou les) circuit(s) d'actionnement 13 est(sont) délimité(s) par la membrane déformable 43 et le premier substrat 41, tandis que le réseau de canaux microfluidiques 5 est délimité par la membrane déformable 43 et le second substrat 45. Ainsi, le réseau de canaux microfluidiques 5 et le(les) circuit(s) d'actionnement 13 sont séparés par la membrane déformable 43 aux niveaux des zones de croisement formant une vanne à chaque croisement.

Le matériau utilisé pour la fabrication de la pompe surfacique est un polymère du type PDMS (Polydiméthylsiloxane) selon par exemple un mélange de dix volumes de polymère pour un volume de réticulant. La technique de fabrication peut être similaire à celle décrite par exemple dans le document « Monolithic Microfabricated Valves and Pumps by Multilayer Soft Lithography », Marc Unger, et al. Science 288, 113 (2000).

Plus particulièrement, la Fig. 7A montre une étape de fabrication du premier substrat 51 par le moulage du polymère sur une empreinte 47 comportant un motif 49 destiné à former le futur circuit microfluidique d'actionnement 13.

Après polymérisation, le premier substrat 51 est démoulé de l'empreinte 47 comme illustré sur la Fig. 7B.

La Fig. 7C montre la réalisation de la membrane par polymérisation d'une fine couche de PDMS étalée à la tournette sur une empreinte 61 comportant un motif 63 destiné à former le futur réseau de canaux microfluidiques 5. A titre d'exemple, l'épaisseur de la membrane est d'environ 15 $\mu m$.

Après polymérisation (Fig. 7D), le premier substrat 51 est assemblé à la membrane 43 et ils sont collés ensemble, par exemple par plasma $O_2$ comme illustré sur la Fig. 7D.

La Fig. 7E montre que la bicouche formée par le premier substrat 51 et la membrane 43, est démoulée de l'empreinte 43, puis assemblée et collée sur un second substrat en PDMS par exemple, par plasma $O_2$ comme illustré sur la Fig. 7F.

Des trous (non représentés) sont réalisés à travers le substrat 45 par poinçonnage avant l'étape d'assemblage et de collage.

En outre, la présente invention vise un système de pompage comportant une pompe surfacique selon l'un quelconque des modes de réalisations précédents.

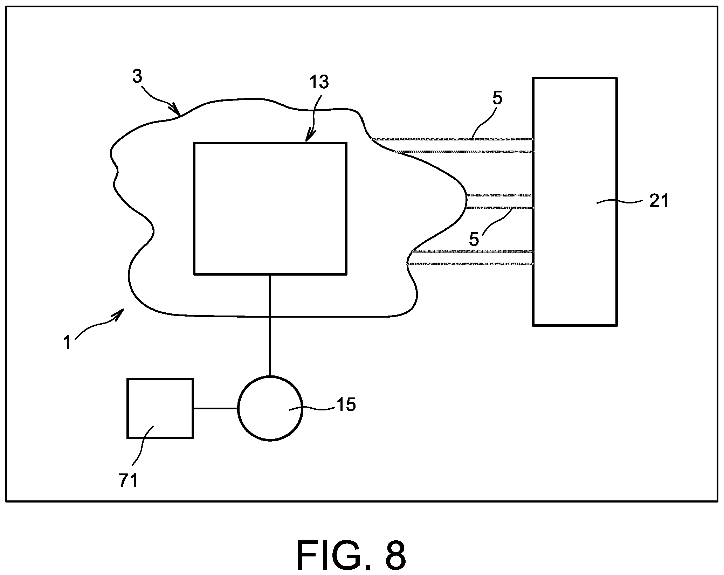

La Fig. 8 illustre de manière très schématique un système de pompage selon un mode de réalisation de l'invention.

Le système de pompage 101 comporte au moins un générateur de pression 15 relié au (ou aux) circuit(s) d'actionnement 13 de la pompe surfacique 1 ainsi qu'un dispositif de commande 71 du générateur de pression 15. Le dispositif de commande 71 comporte par exemple une unité d'alimentation électrique et une unité de traitement destinée à contrôler le générateur de pression 15.

Avantageusement, le système de pompage 101 comporte au moins un puits 21 relié au réseau 3 de canaux microfluidiques 5 de la pompe surfacique.

La présente invention vise également un pansement intégrant une pompe surfacique selon l'un quelconque des modes de réalisations précédents. Le pansement est réalisé en une matière flexible et biocompatible et comporte au moins un puits connecté au réseau de canaux microfluidiques. Le puits est destiné à collecter le fluide d'intérêt pompé par la pompe surfacique.

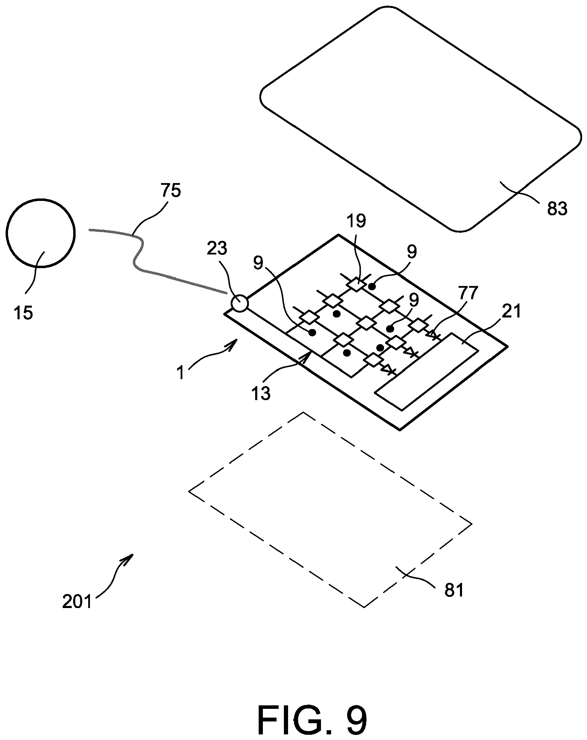

La Fig. 9 illustre de manière schématique un pansement pour plaies exsudatives comportant une pompe surfacique, selon un mode de réalisation préféré de l'invention.

Selon ce mode de réalisation, le pansement 201 comporte un ensemble de canaux de drainage microfluidiques 5 et un circuit microfluidique d'actionnement 13.

L'ensemble de canaux de drainage microfluidiques 5 est muni d'une pluralité d'accès 9 microfluidiques localisés définissant un domaine prédéterminé adapté pour couvrir une plaie sécrétant de l'exsudat.

Le circuit microfluidique d'actionnement 13 comporte un unique port de connexion 23 permettant de réduire l'encombrement du pansement. Ce dernier permet de relier de manière détachable le circuit microfluidique d'actionnement 13 via par exemple un raccordement 75 à un unique générateur de pression 15. Le fait que le circuit microfluidique d'actionnement 13 est directement relié au générateur 15 évite le recours à un pansement 201 étanche, ce qui simplifie sa pose, sa surveillance, et son entretien.

On notera qu'un simple générateur de pression 15 non encombrant et portable suffit pour opérer le drainage selon l'invention. De plus, le générateur de pression 15 n'est jamais en contact avec les exsudats et peut donc être réutilisé en toute sécurité.

Le circuit microfluidique d'actionnement 13 est réalisé de manière à croiser les canaux microfluidiques de drainage 5 sur une pluralité de zones de croisement à membrane déformable formant un réseau de vannes 19.

Les vannes 19 sont adaptées pour engendrer sous un actionnement contrôlé du générateur de pression 15, un micropompage à effet péristaltique de l'exsudat circulant dans les canaux de drainage microfluidiques 5 depuis l'ensemble d'accès 9 microfluidiques vers au moins un puits 21 connecté à l'ensemble de canaux de drainage micro-fluidiques. Ainsi, le drainage de l'exsudat est réalisé efficacement de manière indépendante sur chaque zone parmi une multitude de zones et indépendamment du fait que le taux d'exsudation puisse varier entre les différentes zones.

Avantageusement, ledit au moins un puits 21 est intégré au pansement 201. Ceci facilite la pose du pansement et supprime des connexions à une poubelle externe. Le puits 21 peut être réalisé en un matériau très hydrophile et très absorbant.

Avantageusement, l'ensemble de canaux de drainage microfluidiques 5 et le circuit microfluidique d'actionnement 13 sont réalisés en matière flexible et biocompatible permettant de les déposer directement sur la plaie ou à proximité de cette dernière.

En variante, le pansement peut comporter une couche support 81 sur laquelle sont disposés la pompe surfacique 1 et le ou les puits 21. La couche support 81 est réalisée en un matériau poreux biocompatible (par exemple, en tissu) destiné à être en contact avec la plaie.

En outre, le pansement 201 comporte une couche externe 83 de protection par exemple, en forme de film autocollant pour fixer le pansement 201 sur la plaie et pour protéger la plaie, la pompe surfacique 1, et le ou les puits.

La Fig. 10A illustre de manière schématique un pansement selon un premier mode de réalisation préféré de l'invention.

Selon ce premier mode de réalisation, les accès 9 microfluidiques sont rangés de manière régulière ligne par ligne et colonne par colonne formant une matrice d'accès 9 microfluidiques à $m$ lignes et $n$ colonnes. La position $(i, j)$ de chaque accès 9 microfluidique peut être définie par le numéro de ligne $i$ et le numéro de colonne $j$, avec $i = 1,...,m$ et $j = 1,...,n$. La matrice d'accès microfluidiques occupe une surface rectangulaire destinée à couvrir la plaie.

Par ailleurs, l'ensemble de canaux de drainage micro-fluidiques comporte un ensemble de canaux microfluidiques principaux 51 sensiblement parallèles entre eux ainsi qu'une pluralité de canaux microfluidiques secondaires raccordant les accès 9 microfluidiques aux canaux principaux 51.

Les canaux microfluidiques principaux 51 sont disposés selon des lignes sensiblement parallèles aux lignes de la matrice d'accès microfluidiques. Plus particulièrement, chaque ligne d'accès 9 microfluidiques est encadrée par deux canaux microfluidiques principaux 51. Ainsi, le pansement 201 peut comporter n + 1 lignes de canaux microfluidiques principaux 51 de sorte que chaque ligne d'accès 9 microfluidiques est située entre deux canaux 51 consécutifs.

Avantageusement, les accès 9 microfluidiques disposés sur une ligne sont raccordés de manière alternée via des canaux microfluidiques secondaires 53, avec les deux canaux microfluidiques principaux 51 d'encadrement. Par exemple, les accès 9 microfluidiques sont reliés avec l'un ou l'autre des canaux microfluidiques principaux 51 d'encadrement, selon la parité de leurs indices colonne.

Avantageusement, l'ensemble d'accès 9 microfluidiques forme une relation bijective avec l'ensemble de canaux microfluidiques secondaires 53. Autrement dit, chaque accès 9 microfluidique est connecté à un canal fluidique principal 51 via un et un seul canal fluidique secondaire 53.

Selon ce mode de réalisation, l'ensemble de canaux 51 est constitué de deux sous-ensembles distincts de canaux microfluidiques principaux 51a, 51b. Un premier sous-ensemble de canaux principaux 51a est associé à la première moitié des colonnes ($j = 1,...,\frac{n}{2}$) de la matrice d'accès 9 microfluidiques et un second sous-ensemble de canaux principaux 51b est associé à la seconde moitié des colonnes ($j = \frac{n}{2} + 1,....,n$) de la matrice d'accès 9 microfluidiques.

Le premier sous-ensemble de canaux microfluidiques principaux 51a est connecté à un premier puits 21a. Plus particulièrement, le premier puits 51a est connecté aux extrémités des canaux microfluidiques principaux 51a qui sont du côté de la première colonne ($j = 1$) des accès 9 microfluidiques. De même, le second sous-ensemble de canaux microfluidiques principaux 51b est connecté à un second puits 21b. Ce dernier est connecté aux extrémités des canaux microfluidiques principaux 51b qui sont du côté de la dernière colonne ($j = n$) des accès 9 microfluidiques. Les autres extrémités des canaux microfluidiques principaux 51a, 51b des premier et second sous-ensembles sont fermées.

A titre d'exemple, le canal microfluidique principal 51a situé entre les lignes $i$ et $i + 1$ et appartenant au premier sous-ensemble de canaux est relié de manière alternée aux accès 9 microfluidiques d'indices $(i, 2k - 1)$ et $(i + 1,2k)$ avec $k = 1,...,\frac{n}{4}$. De même, le canal microfluidique principal 51b situé entre les lignes $i$ et $i + 1$ et appartenant au second sous-ensemble est relié de manière alterné aux accès 9 microfluidiques d'indices $(i, 2k - 1)$ et $(i + 1,2k)$ avec $k = \frac{n}{4} + 1,...., \frac{n}{2}$.

Par ailleurs, selon ce premier mode de réalisation, le circuit microfluidique d'actionnement est formé d'un canal microfluidique d'actionnement 113 en forme de serpentin. Les deux extrémités 25a, 25b du serpentin d'actionnement 113 sont fermées tandis qu'un point intermédiaire 25c entre ces deux extrémités est connecté via un unique port de connexion 23 à un générateur de pression 15.

Le serpentin d'actionnement 113 est configuré pour croiser chaque canal microfluidique principal 51a, 51b ainsi que chaque canal microfluidique secondaire 53a, 53b au moins une fois entre chaque deux accès 9 microfluidiques consécutifs situés sur une même ligne.

Plus particulièrement, le serpentin d'actionnement 113 est un canal microfluidique en forme de créneaux périodiques avec des segments verticaux d'actionnement 91 (sensiblement perpendiculaires aux lignes des accès 9 microfluidiques) et des segments horizontaux 93. Au moins un segment vertical d'actionnement 91 est disposé entre deux colonnes consécutives d'accès 9 microfluidiques. En outre, les deux segments verticaux d'actionnement 91 du milieu croisant respectivement les premier et second sous-ensembles de canaux microfluidiques 51a, 51b, sont connectés au générateur de pression 51.

Avantageusement, comme dans l'exemple de la Fig. 10A, trois segments verticaux d'actionnement 91 ou plus sont disposés entre chaque deux colonnes consécutives d'accès 9 microfluidiques. Ainsi, chaque accès 9 microfluidique est associé à au moins trois vannes de sorte que l'exsudat pénétrant dans chaque accès microfluidique est pompé de manière péristaltique vers le puits 21a, 21b correspondant sans aucun risque de refoulement. Les vannes sont disposées aux zones de croisement entre les segments verticaux d'actionnement 91 et les canaux microfluidiques principaux 51a, 51b et secondaires 53a, 53b et ne sont pas représentées sur la Fig. 10A par souci de simplification. Les flèches 29a et 29b indiquent le sens de propagation d'ouverture/fermeture des vannes induisant le drainage de l'exsudat vers les puits latéraux 21a et 21b.

La Fig. 10B illustre de manière schématique un pansement selon un deuxième mode de réalisation préféré de l'invention.

Ce deuxième mode de réalisation se distingue de celui de la Fig. 10A uniquement par le fait que le circuit microfluidique d'actionnement comporte un ensemble de canaux d'actionnement en forme de peigne sensiblement perpendiculaires aux lignes des accès microfluidiques, et définissant des éléments capacitifs. Cet ensemble de canaux d'actionnement est constitué de deux groupes de canaux d'actionnement. Un premier groupe de canaux d'actionnement 33a est associé au premier sous-ensemble de canaux principaux 51a et un second groupe de canaux d'actionnement 33b est associé au second sous-ensemble de canaux principaux 51b.

Les premier et second groupes de canaux d'actionnement 33a en forme de peigne sont raccordés à un canal de raccordement 35 sensiblement parallèles aux lignes des accès microfluidiques. En variantes, les premier et second groupes de canaux d'actionnement 33a, 33b peuvent être raccordés respectivement à des premier et second canaux de raccordement 35 distincts (non représentés). Les vannes sont localisées sur les premier et second groupes de canaux d'actionnement 33a, 33b.

Comme dans l'exemple précédent, au moins un canal vertical d'actionnement 33a, 33b est disposé entre chaque deux colonnes consécutives d'accès 9 microfluidiques et les deux canaux verticaux d'actionnement du milieu croisant respectivement les premier et second sous-ensembles de canaux microfluidiques, sont connectés au générateur de pression 15.

Avantageusement, trois canaux verticaux d'actionnement 33a ou plus sont disposés entre chaque deux colonnes consécutives d'accès 9 microfluidiques de sorte que chaque accès microfluidique soit associé à au moins trois vannes.

Enfin, le pansement selon l'invention peut comporter une pompe surfacique selon d'autres modes de réalisation comme par exemple celui de la Fig. 6.

**Revendications**

**1.** Pompe surfacique comportant un réseau (3) de canaux microfluidiques (5) et des moyens de pompage (7), **caractérisée en ce que** ledit réseau de canaux microfluidiques comprend :

- un ensemble d'accès (9) microfluidiques distribués dans un domaine prédéterminé (11) adapté pour couvrir une source surfacique d'un fluide d'intérêt,
- et **en ce que** lesdits moyens de pompage comportent au moins un circuit microfluidique d'actionnement (11) croisant ledit réseau de canaux microfluidiques sur une pluralité de zones de croisement (17) à membrane déformable formant un ensemble de vannes (19),
- ledit ensemble de vannes étant adapté pour engendrer un pompage à effet péristaltique dudit fluide d'intérêt circulant dans ledit réseau de canaux microfluidiques depuis ledit ensemble d'accès microfluidiques vers au moins un puits (21) connecté audit réseau de canaux microfluidiques.

**2.** Pompe surfacique selon la revendication 1, **caractérisée en ce que** ledit réseau de canaux microfluidiques comporte un ensemble de canaux de drainage microfluidiques (5) distincts muni dudit ensemble d'accès microfluidiques, chaque canal de drainage microfluidique étant associé à un groupe de vannes déterminé, et **en ce qu'**il comporte un unique circuit microfluidique d'actionnement (13) adapté pour être relié à un unique générateur de pression (15), ledit circuit d'actionnement étant constitué des éléments fluidiques résistifs et capacitifs définissant un retard de temps de réponse entre deux vannes successives appartenant à un groupe de vannes, ledit retard de temps de réponse étant configuré pour engendrer sous une action dudit générateur de pression, un actionnement séquentiel des vannes.

3. Pompe surfacique selon la revendication 2, **caractérisée en ce que** ledit ensemble de canaux de drainage micro-fluidiques comporte un ensemble de canaux microfluidiques principaux (51) et une pluralité de canaux microfluidiques secondaires (53), chaque canal de drainage microfluidique comportant un canal microfluidique principal et un ensemble de canaux microfluidiques secondaires raccordant le canal principal aux accès (9) microfluidiques associés.

4. Pompe surfacique selon la revendication 2 ou 3, **caractérisée en ce que** ledit circuit microfluidique d'actionnement est formé d'un canal microfluidique d'actionnement en forme de serpentin, dit serpentin d'actionnement (113), présentant une longueur caractéristique $L$ entre deux vannes successives appartenant à un même groupe de vannes, une largeur $w$, une hauteur $h$, et un paramètre de déformation $A$, vérifiant la double inégalité suivante :

$$1 \times 10^{-3} \leq A \frac{wL^2}{h^3} \leq 10 \text{ et de préférence } 0{,}01 \leq A \frac{wL^2}{h^3} \leq 1.$$

5. Pompe surfacique selon la revendication 2 ou 3, **caractérisée en ce que** ledit circuit microfluidique d'actionnement comporte des canaux d'actionnement (33) en forme de peigne définissant des éléments capacitifs, raccordés à un canal de raccordement (35) définissant un élément résistif, les vannes (19) étant localisées sur lesdits canaux d'actionnement.

6. Pompe surfacique selon l'une quelconque des revendications 3 à 5, **caractérisée en ce que** des extrémités avals (27a) desdits canaux microfluidiques principaux sont connectées à un même puits, les autres extrémités (27b) étant fermées.

7. Pompe surfacique selon l'une quelconque des revendications 3 à 5, **caractérisée en ce que** l'ensemble de canaux microfluidiques principaux est constitué des premier et second sous-ensembles distincts de canaux microfluidiques principaux (51a, 51b), le premier sous-ensemble de canaux microfluidiques principaux étant connecté à un premier puits (21a) et le second sous-ensemble de canaux microfluidiques principaux étant connecté à un second puits (21b).

8. Pompe surfacique selon l'une quelconque des revendications 2 à 7, **caractérisée en ce que** ledit générateur de pression est configuré pour générer des créneaux de pression pour actionner de manière cyclique les vannes appartenant à chaque groupe de vannes.

9. Pompe surfacique selon la revendication 1, **caractérisée en ce que** ledit réseau de canaux microfluidiques comporte un canal microfluidique principal (151) en forme de spirale raccordé via des canaux microfluidiques secondaires (153) audit ensemble d'accès (9) microfluidiques, et **en ce qu'**elle comporte au moins trois circuits microfluidiques d'actionnement (313a, 313b, 313c) distincts adaptés pour être reliés à au moins trois générateurs de pression distincts, chaque circuit d'actionnement étant constitué d'un canal d'actionnement en forme de spirale, lesdits au moins trois canaux d'actionnement coopérant avec chaque canal microfluidique secondaire pour former au moins trois vannes (19a, 19b, 19c) configurées pour être actionnées de manière séquentielle par lesdits au moins trois générateurs de pression.

10. Pompe surfacique selon l'une quelconque des revendications précédentes, **caractérisée en ce qu'**elle comporte un premier substrat (41), une membrane déformable (43), et un second substrat (45), ledit au moins un circuit d'actionnement (13) étant délimité par ladite membrane déformable et ledit premier substrat, ledit réseau de canaux microfluidiques (5) étant délimité par ladite membrane déformable et ledit second substrat, ledit réseau de canaux microfluidiques et ledit au moins un circuit d'actionnement étant alors séparés par ladite membrane déformable aux niveaux des zones de croisement formant ainsi une vanne à chaque croisement.

11. Pompe surfacique selon l'une quelconque des revendications précédentes, **caractérisée en ce qu'**elle comporte un nombre déterminé de vannes compris entre environ 10 et 1000.

12. Système de pompage comportant une pompe surfacique selon l'une quelconque des revendications précédentes, comportant en outre au moins un générateur de pression relié audit au moins un circuit d'actionnement de ladite pompe surfacique.

13. Pansement comportant une pompe surfacique selon l'une quelconque des revendications 1 à 11, **caractérisé en**

# EP 3 040 092 B1

**ce qu'**il comporte au moins un puits connecté à ladite pompe surfacique, ledit au moins un puits étant destiné à collecter le fluide d'intérêt pompé par la pompe surfacique.

14. Pansement pour des plaies exsudatives, **caractérisé en ce qu'**il comporte un ensemble de canaux de drainage microfluidiques (5) munis d'une pluralité d'accès (9) microfluidiques définissant un domaine prédéterminé adapté pour couvrir une plaie sécrétant de l'exsudat, un circuit microfluidique d'actionnement (13) adapté pour être relié à un unique générateur de pression (15), ledit circuit microfluidique d'actionnement croisant lesdits canaux de drainage microfluidiques sur une pluralité de zones de croisement à membrane déformable formant un ensemble de vannes (19) adapté pour engendrer sous un actionnement dudit générateur de pression, un pompage à effet péristaltique de l'exsudat circulant dans lesdits canaux de drainage microfluidiques depuis ledit ensemble d'accès microfluidiques vers au moins un puits (21) connecté audit ensemble de canaux de drainage microfluidiques.

15. Pansement selon la revendication 14, **caractérisé en ce que** ledit circuit microfluidique d'actionnement est formé d'un canal microfluidique d'actionnement en forme de serpentin.

16. Pansement selon la revendication 14, **caractérisé en ce que** ledit circuit microfluidique d'actionnement comporte des canaux d'actionnement (33a, 33b) en forme de peigne définissant des éléments capacitifs, raccordés à au moins un canal de raccordement (35) définissant un élément résistif, les vannes étant localisées sur lesdits canaux d'actionnement.

17. Pansement selon la revendication 16, **caractérisé en ce que** ledit ensemble de canaux de drainage microfluidiques comporte un ensemble de canaux microfluidiques principaux (51) et une pluralité de canaux microfluidiques secondaires (53), chaque canal de drainage microfluidique comportant un canal microfluidique principal et un ensemble de canaux microfluidiques secondaires raccordant le canal principal aux accès microfluidiques associés.

18. Pansement selon la revendication 17, **caractérisé en ce qu'**il comporte des premier et deuxième puits (21a, 21b) pour collecter les exsudats sécrétés par la plaie, et **en ce que** ledit ensemble de canaux microfluidiques principaux est constitué des premier et second sous-ensembles distincts de canaux microfluidiques principaux (51a, 51b), le premier sous-ensemble de canaux microfluidiques principaux étant connecté au premier puits (21a) et le second sous-ensemble de canaux microfluidiques principaux étant connecté au second puits (21b).

19. Pansement selon l'une quelconque des revendications 14 à 18, **caractérisé en ce que** l'ensemble de canaux de drainage microfluidiques et le canal fluidique d'actionnement sont réalisés en matière biocompatible.

20. Pansement selon l'une quelconque des revendications 14 à 19, **caractérisé en ce qu'**il comporte une couche support (81) accueillant l'ensemble de canaux de drainage microfluidiques et le canal fluidique d'actionnement, ladite couche support étant formée d'un matériau poreux biocompatible destiné à être en contact avec la plaie.

21. Pansement selon l'une quelconque des revendications 14 à 20, **caractérisé en ce qu'**il comporte une couche externe (83) autocollante.

## Patentansprüche

1. Flächige Pumpe mit einem Netz (3) aus mikrofluidischen Kanälen (5) und Pumpeinrichtungen (7), **dadurch gekennzeichnet, dass** das Netz aus mikrofluidischen Kanälen enthält:

   - eine Anordnung aus mikrofluidischen Zugängen (9), die in einem vorbestimmten Bereich (11) verteilt sind, der dazu ausgelegt ist, eine flächige Quelle eines betreffenden, interessierenden Fluids zu bedecken,
   - und dass die Pumpeinrichtungen zumindest eine mikrofluidische Betätigungsschaltung (11) enthalten, welche das Netz aus mikrofluidischen Kanälen über eine Mehrzahl von Kreuzungsbereichen (17) mit verformbarer Membran kreuzt, die eine Anordnung von Ventileinrichtungen (19) bilden,
   - wobei die Anordnung von Ventileinrichtungen dazu ausgelegt ist, eine peristaltische Pumpwirkung für das betreffende Fluid zu erzeugen, das in dem Netz aus mikrofluidischen Kanälen von der Anordnung aus mikrofluidischen Zugängen hin zu zumindest einer Mulde (21) strömt, die mit dem Netz aus mikrofluidischen Kanälen verbunden ist.

2. Flächige Pumpe nach Anspruch 1, **dadurch gekennzeichnet, dass** das Netz aus mikrofluidischen Kanälen eine Anordnung von separaten mikrofluidischen Drainagekanälen (5) enthält, die mit der Anordnung von mikrofluidischen Zugängen versehen ist, wobei jedem mikrofluidischen Drainagekanal eine bestimmte Gruppe von Ventileinrichtungen zugeordnet ist, und dass sie eine einzelne mikrofluidische Betätigungsschaltung (13) enthält, die dazu ausgelegt ist, mit einem einzelnen Druckerzeuger (15) verbunden zu werden, wobei die Betätigungsschaltung aus fluidischen resistiven und kapazitiven Elementen besteht, die eine Ansprechzeitverzögerung zwischen zwei aufeinanderfolgenden Ventileinrichtungen definiert, die zu einer Gruppe von Ventileinrichtungen gehören, wobei die Ansprechzeitverzögerung dazu konfiguriert ist, unter der Wirkung des Druckerzeugers eine sequentielle Betätigung der Ventileinrichtungen zu bewirken.

3. Flächige Pumpe nach Anspruch 2, **dadurch gekennzeichnet, dass** die Anordnung von mikrofluidischen Drainagekanälen eine Anordnung von mikrofluidischen Hauptkanälen (51) und eine Mehrzahl von mikrofluidischen Nebenkanälen (53) enthält, wobei jeder mikrofluidische Drainagekanal einen mikrofluidischen Hauptkanal und eine Anordnung von mikrofluidischen Nebenkanälen enthält, welche den Hauptkanal mit den zugeordneten mikrofluidischen Zugängen (9) verbindet.

4. Flächige Pumpe nach Anspruch 2 oder 3, **dadurch gekennzeichnet, dass** die mikrofluidische Betätigungsschaltung aus einem schlangenförmigen mikrofluidischen Betätigungskanal, Betätigungsschlange (113) genannt, gebildet ist, die eine charakteristische Länge $L$ zwischen zwei aufeinanderfolgenden, einer gleichen Gruppe von Ventileinrichtungen zugehörigen Ventileinrichtungen, eine Breite $w$, eine Höhe $h$ und einen Verformungsparameter $A$ aufweist, welche die nachfolgende, zweifache Ungleichung erfüllen:

$$1 \times 10^{-3} \le A\,\frac{wL^2}{h^3} \le 10 \text{ und vorzugsweise } 0{,}01 \le A\,\frac{wL^2}{h^3} \le 1.$$

5. Flächige Pumpe nach Anspruch 2 oder 3, **dadurch gekennzeichnet, dass** die mikrofluidische Betätigungsschaltung kammförmige Betätigungskanäle (33) enthält, die kapazitive Elemente definieren, die mit einem Verbindungskanal (35) verbunden sind, der ein resistives Element definiert, wobei die Ventileinrichtungen (19) an den Betätigungskanälen angeordnet sind.

6. Flächige Pumpe nach einem der Ansprüche 3 bis 5, **dadurch gekennzeichnet, dass** stromabwärtige Enden (27a) der mikrofluidischen Hauptkanäle mit einer gleichen Mulde verbunden sind, wobei die weiteren Enden (27b) verschlossen sind.

7. Flächige Pumpe nach einem der Ansprüche 3 bis 5, **dadurch gekennzeichnet, dass** die Anordnung aus mikrofluidischen Hauptkanälen aus einer ersten und einer zweiten separaten Untereinheit von mikrofluidischen Hauptkanälen (51a, 51b) besteht, wobei die erste Untereinheit von mikrofluidischen Hauptkanälen mit einer ersten Mulde (21a) verbunden ist und die zweite Untereinheit von mikrofluidischen Hauptkanälen mit einer zweiten Mulde (21b) verbunden ist.

8. Flächige Pumpe nach einem der Ansprüche 2 bis 7, **dadurch gekennzeichnet, dass** der Druckerzeuger dazu konfiguriert ist, Drucklücken zu erzeugen, um die zu jeder Gruppe von Ventileinrichtungen gehörenden Ventileinrichtungen zyklisch zu betätigen.

9. Flächige Pumpe nach Anspruch 1, **dadurch gekennzeichnet, dass** das Netz aus mikrofluidischen Kanälen einen spiralförmigen mikrofluidischen Hauptkanal (151) enthält, der über mikrofluidische Nebenkanäle (153) mit der Anordnung von mikrofluidischen Zugängen (9) verbunden ist, und dass sie zumindest drei separate mikrofluidische Betätigungsschaltungen (313a, 313b, 313c) enthält, die dazu ausgelegt sind, mit zumindest drei separaten Druckerzeugern verbunden zu werden, wobei jede Betätigungsschaltung aus einem spiralförmigen Betätigungskanal besteht, wobei die zumindest drei Betätigungskanäle mit jedem mikrofluidischen Nebenkanal zusammenwirken, um zumindest drei Ventileinrichtungen (19a, 19b, 19c) zu bilden, die dazu konfiguriert sind, über die zumindest drei Druckerzeuger sequentiell betätigt zu werden.

10. Flächige Pumpe nach einem der vorangehenden Ansprüche, **dadurch gekennzeichnet, dass** sie ein erstes Substrat (41), eine verformbare Membran (43) und ein zweites Substrat (45) enthält, wobei die zumindest eine Betätigungsschaltung (13) von der verformbaren Membran und dem ersten Substrat eingegrenzt wird, wobei das Netz aus mikrofluidischen Kanälen (5) von der verformbaren Membran und dem zweiten Substrat eingegrenzt wird, wobei das Netz aus mikrofluidischen Kanälen und die zumindest eine Betätigungsschaltung dann durch die verformbare Membran im Bereich der Kreuzungsbereiche getrennt sind, die somit an jedem Kreuzungspunkt eine Ventileinrichtung bilden.

11. Flächige Pumpe nach einem der vorangehenden Ansprüche, **dadurch gekennzeichnet, dass** sie eine bestimmte Anzahl von Ventileinrichtungen zwischen etwa 10 und 1000 enthält.

12. Pumpsystem mit einer flächigen Pumpe nach einem der vorangehenden Ansprüche, ferner enthaltend zumindest einen Druckerzeuger, der mit der zumindest einen Betätigungsschaltung der flächigen Pumpe verbunden ist.

13. Verband mit einer flächigen Pumpe nach einem der Ansprüche 1 bis 11, **dadurch gekennzeichnet, dass** er zumindest eine Mulde enthält, die mit der flächigen Pumpe verbunden ist, wobei die zumindest eine Mulde dazu bestimmt ist, das von der flächigen Pumpe gepumpte betreffende Fluid zu sammeln.

14. Verband für exsudierende Wunden, **dadurch gekennzeichnet, dass** er eine Anordnung von mikrofluidischen Drainagekanälen (5) enthält, die mit einer Mehrzahl von mikrofluidischen Zugängen (9) versehen sind, die einen vorbestimmten Bereich definieren, der dazu ausgelegt ist, eine Exsudat absondernde Wunde zu bedecken, sowie eine mikrofluidische Betätigungsschaltung (13), die dazu ausgelegt ist, mit einem einzelnen Druckerzeuger (15) verbunden zu werden, wobei die mikrofluidische Betätigungsschaltung die mikrofluidischen Drainagekanäle an einer Mehrzahl von Kreuzungsbereichen mit verformbarer Membran kreuzt, die eine Anordnung von Ventileinrichtungen (19) bilden, die dazu ausgelegt ist, unter einer Betätigung des Druckerzeugers eine peristaltische Pumpwirkung für das Exsudat zu erzeugen, das in den mikrofluidischen Drainagekanälen von der Anordnung aus mikrofluidischen Zugängen hin zu zumindest einer Mulde (21) strömt, die mit der Anordnung aus mikrofluidischen Drainagekanälen verbunden ist.

15. Verband nach Anspruch 14, **dadurch gekennzeichnet, dass** die mikrofluidische Betätigungsschaltung aus einem schlangenförmigen mikrofluidischen Betätigungskanal gebildet ist.

16. Verband nach Anspruch 14, **dadurch gekennzeichnet, dass** die mikrofluidische Betätigungsschaltung kammförmige Betätigungskanäle (33a, 33b) enthält, die kapazitive Elemente definieren, die mit zumindest einem Verbindungskanal (35) verbunden sind, der ein resistives Element definiert, wobei die Ventileinrichtungen an den Betätigungskanälen angeordnet sind.

17. Verband nach Anspruch 16, **dadurch gekennzeichnet, dass** die Anordnung von mikrofluidischen Drainagekanälen einen Anordnung von mikrofluidischen Hauptkanälen (51) und eine Mehrzahl von mikrofluidischen Nebenkanälen (53) enthält, wobei jeder mikrofluidische Drainagekanal einen mikrofluidischen Hauptkanal und eine Anordnung von mikrofluidischen Nebenkanälen enthält, die den Hauptkanal mit den zugeordneten mikrofluidischen Zugängen verbinden.

18. Verband nach Anspruch 17, **dadurch gekennzeichnet, dass** er eine erste und eine zweite Mulde (31a, 31b) zum Sammeln von aus der Wunde abgesonderten Exsudaten enthält und dass die Anordnung von mikrofluidischen Hauptkanälen aus einer ersten und einer zweiten separaten Untereinheit von mikrofluidischen Hauptkanälen (51a, 51b) besteht, wobei die erste Untereinheit von mikrofluidischen Hauptkanälen mit der ersten Mulde (21a) verbunden ist und die zweite Untereinheit von mikrofluidischen Hauptkanälen mit der zweiten Mulde (21b) verbunden ist.

19. Verband nach einem der Ansprüche 14 bis 18, **dadurch gekennzeichnet, dass** die Anordnung von mikrofluidischen Drainagekanälen und der fluidische Betätigungskanal aus einem biokompatiblen Material hergestellt sind.

20. Verband nach einem der Ansprüche 14 bis 19, **dadurch gekennzeichnet, dass** er eine Trägerschicht (81) enthält, welche die Anordnung von mikrofluidischen Drainagekanälen und den fluidischen Betätigungskanal aufnimmt, wobei die Trägerschicht aus einem biokompatiblen porösen Material ausgebildet ist, das dazu bestimmt ist, mit der Wunde in Kontakt zu stehen.

21. Verband nach einem der Ansprüche 14 bis 20, **dadurch gekennzeichnet, dass** er eine äußere Selbstklebeschicht (83) enthält.

**Claims**

1. Surface pump comprising a network (3) of microfluidic channels (5) and pumping means (7), **characterised in that** said network of microfluidic channels includes:

   - a set of microfluidic accesses (9) distributed in a predetermined domain (11) adapted to covering a surface source of a fluid of interest,
   - and **in that** said pumping means comprise at least one microfluidic actuating circuit (11) intersecting said network of microfluidic channels on a plurality of deformable membrane intersecting zones (17) forming a set of valves (19),
   - said set of valves being adapted to causing a peristaltic effect pumping of said fluid of interest circulating in said network of microfluidic channels from said set of microfluidic accesses to at least one sink (21) connected to said network of microfluidic channels.

2. Surface pump according to claim 1, **characterised in that** said network of microfluidic channels comprises a set of separate microfluidic drainage channels (5) provided with said set of microfluidic accesses, each microfluidic drainage channel being associated with a determined group of valves, and **in that** it comprises a single microfluidic actuating circuit (13) adapted to being connected to a single pressure generator (15), said actuating circuit being constituted of resistive and capacitive fluidic elements defining a response time delay between two successive valves belonging to a group of valves, said response time delay being configured to cause, under an action of said pressure generator, a sequential actuation of the valves.

3. Surface pump according to claim 2, **characterised in that** said set of microfluidic drainage channels comprises a set of main microfluidic channels (51) and a plurality of secondary microfluidic channels (53), each microfluidic drainage channel comprising a main microfluidic channel and a set of secondary microfluidic channels connecting the main channel to the associated microfluidic accesses (9).

4. Surface pump according to claim 2 or 3, **characterised in that** said microfluidic actuating circuit is formed of a coil-shaped microfluidic actuating channel, called actuating coil (113), having a characteristic length $L$ between two successive valves belonging to a same group of valves, a width $w$, a height $h$, and a deformation parameter $A$,

   bearing out the following double inequality: $1 \times 10^{-3} \leq A \frac{wL^2}{h^3} \leq 10$ and preferably $0.01 \leq A \frac{wL^2}{h^3} \leq 1$.

5. Surface pump according to claim 2 or 3, **characterised in that** said microfluidic actuating circuit comprises comb-shaped actuating channels (33) defining capacitive elements, connected to a connection channel (35) defining a resistive element, the valves (19) being located on said actuating channels.

6. Surface pump according to anyone of claims 3 t 5, **characterised in that** the downstream ends (27a) of said main microfluidic channels are connected to a same sink, the other ends (27b) being closed.

7. Surface pump according to anyone of claims 3 to 5, **characterised in that** the set of main microfluidic channels is constituted of separate first and second sub-assemblies of main microfluidic channels (51a, 51b), the first sub-assembly of main microfluidic channels being connected to a first sink (21a) and the second sub-assembly of main microfluidic channels being connected to a second sink (21b).

8. Surface pump according to anyone of claims 2 to 7, **characterised in that** said pressure generator is configured to generate pressure slots to actuate in a cyclical manner the valves belonging to each group of valves.

9. Surface pump according to claim 1, **characterised in that** said network of microfluidic channels comprises a spiral-shaped main microfluidic channel (151) connected via secondary microfluidic channels (153) to said set of microfluidic accesses (9), and **in that** it comprises at least three separate microfluidic actuating circuits (313a, 313b, 313c) adapted to be connected to at least three separate pressure generators, each actuating circuit being constituted of a spiral-shaped actuating channel, said at least three actuating channels cooperating with each secondary microfluidic channel to form at least three valves (19a, 19b, 19c) configured to be actuated sequentially by said at least three pressure generators.

10. Surface pump according to anyone of the preceding claims, **characterised in that** it comprises a first substrate (41), a deformable membrane (43), and a second substrate (45), said at least one actuating circuit (13) being delimited by said deformable membrane and said first substrate, said network of microfluidic channels (5) being delimited by said deformable membrane and said second substrate, said network of microfluidic channels and said at least one actuating circuit then being separated by said deformable membrane at the levels of the intersecting zones thus forming a valve at each intersection.

11. Surface pump according to anyone of the preceding claims, **characterised in that** it comprises a determined number of valves comprised between around 10 and 1000.

12. Pumping system comprising a surface pump according to anyone of the preceding claims, further comprising at least one pressure generator connected to said at least one actuating circuit of said surface pump.

13. Dressing comprising a surface pump according to anyone of claims 1 to 11, **characterised in that** it comprises at least one sink connected to said surface pump, said at least one sink being intended to collect the fluid of interest pumped by the surface pump.

14. Dressing for exudative wounds, **characterised in that** it comprises a set of microfluidic drainage channels (5) provided with a plurality of microfluidic accesses (9) defining a predetermined domain adapted to covering a wound secreting exudate, a microfluidic actuating circuit (13) adapted to being connected to a single pressure generator (15), said microfluidic actuating circuit intersecting said microfluidic drainage channels on a plurality of deformable membrane intersecting zones forming a set of valves (19) adapted to causing, under an actuation of said pressure generator, a peristaltic effect pumping of the exudate circulating in said microfluidic drainage channels from said set of microfluidic accesses to at least one sink (21) connected to said set of microfluidic drainage channels.

15. Dressing according to claim 14, **characterised in that** said microfluidic actuating circuit is formed of a coil-shaped microfluidic actuating channel.

16. Dressing according to claim 14, **characterised in that** said microfluidic actuating circuit comprises comb-shaped actuating channels (33a, 33b) defining capacitive elements, connected to at least one connection channel (35) defining a resistive element, the valves being located on said actuating channels.

17. Dressing according to claim 16, **characterised in that** said set of microfluidic drainage channels comprises a set of main microfluidic channels (51) and a plurality of secondary microfluidic channels (53), each microfluidic drainage channel comprising a main microfluidic channel and a set of secondary microfluidic channels connecting the main channel to the associated microfluidic accesses.

18. Dressing according to claim 17, **characterised in that** it comprises first and second sinks (21a, 21b) for collecting the exudates secreted by the wound, and **in that** said set of main microfluidic channels is constituted of separate first and second sub-assemblies of main microfluidic channels (51a, 51b), the first sub-assembly of main microfluidic channels being connected to the first sink (21a) and the second sub-assembly of main microfluidic channels being connected to the second sink (21b).

19. Dressing according to anyone of claims 14 to 18, **characterised in that** the set of microfluidic drainage channels and the actuating fluidic channel are made of biocompatible material.

20. Dressing according to anyone of claims 14 to 19, **characterised in that** it comprises a support layer (81) receiving the set of microfluidic drainage channels and the actuating fluidic channel, said support layer being formed of a biocompatible porous material intended to be in contact with the wound.

21. Dressing according to anyone of claims 14 to 20, **characterised in that** it comprises an external self-adhesive layer (83).

FIG. 1

FIG. 2

FIG. 3

FIG. 4

FIG. 6

FIG. 5

49

47

## FIG. 7A

51

13

## FIG. 7B

63

61

## FIG. 7C

13

51

63

61

## FIG. 7D

13

41

43

5

## FIG. 7E

13

41

43

45

5

## FIG. 7F

FIG. 8

EP 3 040 092 B1

FIG. 9

FIG. 10A

EP 3 040 092 B1

FIG. 10B

EP 3 040 092 B1

## RÉFÉRENCES CITÉES DANS LA DESCRIPTION

**Documents brevets cités dans la description**

- WO 03086232 A
- WO 02082047 A2
- US 2008275409 A1
- FR 2974598

**Littérature non-brevet citée dans la description**

- **MARC UNGER et al.** Monolithic microfabricated Valves and Pumps by Multilayer Soft Lithography. *Science,* 2000, vol. 288, 113
- **MARC UNGER et al.** Monolithic Microfabricated Valves and Pumps by Multilayer Soft Lithography. *Science,* 2000, vol. 288, 113